# EUROPEAN PATENT SPECIFICATION

(11) **EP 4 649 943 B1**
(45) Date of publication and mention of the grant of the patent: **25.11.2026**
(21) Application number: 24176658.3
(22) Date of filing: 17.05.2024
(51) Int. Cl.: A61K 9/20, A61K 31/4458, A61K 47/14

(54) **REDUCED NITROSAMINE FORMATION IN PHARMACEUTICAL COMPOSITIONS COMPRISING METHYLPHENIDATE**
REDUZIERTE NITROSAMINBILDUNG IN PHARMAZEUTISCHEN ZUSAMMENSETZUNGEN MIT METHYLPHENIDAT
FORMATION RÉDUITE DE NITROSAMINE DANS DES COMPOSITIONS PHARMACEUTIQUES COMPRENANT DU MÉTHYLPHÉNIDATE

(43) Date of publication of application: 19.11.2025
(73) Proprietor: Medice Arzneimittel Pütter GmbH & Co. KG, 58638 Iserlohn (DE)
(72) Inventor: KRUG, Martin, 58099 Hagen (DE); BUSS, Uwe, 48167 Muenster (DE); BALLMANN, Christina, 48167 Muenster (DE); AMMER, Richard Markus, 58644 Iserlohn (DE)
(74) Representative: Isarpatent

(56) References cited:
- WO-A1-2005/035000
- US-A1- 2020 000 791

## Description

### Field of the invention

The present invention relates to a pharmaceutical composition containing methylphenidate and having a low nitrosamine concentration. The present invention also relates to a method of producing said pharmaceutical composition and to its use in the treatment of Attention Deficit Disorder. Further, the present invention relates to the use of an ascorbyl fatty acid ester for reducing nitrosamine formation in compositions comprising methylphenidate.

### Background of the invention

Nitrosamines are classified as probable human carcinogens. In 2018, nitrosamines were found in a number of blood pressure medicines known as "sartans". Investigations into the origin of this contamination revealed that many active pharmaceutical ingredients (APIs) and drug products are contaminated with nitrosamines. Nitrosamines are usually formed by a reaction of a secondary or tertiary amine with a nitrosating agent, which is typically derived from nitrite. Since such amines are a common structural motif in APIs and excipients are often contaminated with nitrites, nitrosamines can form in many pharmaceutical compositions. See, e.g., R. Boetzel et al., Journal of Pharmaceutical Sciences (2023), 112, 1615-1624.

Accordingly, the reduction of nitrosamine formation is the subject of much research and development of new formulations of pharmaceutical compositions. For example, CN 115 721 724 A proposes to reduce nitrosamine formation from secondary amines by adding pharmaceutically acceptable acids. However, nitrosamine formation was only investigated using varenicline and it remains unclear whether the concept is transferable to other active ingredients and compositions. Patent document WO 2005/035000 A1 discloses stable methylphenidate solutions comprising methylphenidate and at least one pharmaceutically acceptable organic acid.

Attention Deficit Disorder (ADD), a commonly diagnosed nervous system illness in children, is generally treated with methylphenidate hydrochloride (available commercially as, e.g., Ritalin^{®}). Symptoms of ADD include distractibility and impulsivity. A related disorder, termed Attention Deficit Hyperactivity Disorder (ADHD), is further characterized by symptoms of hyperactivity, and is also treated with methylphenidate hydrochloride.

As methylphenidate contains the structural motif of a secondary amine, the formation of nitrosamines in pharmaceutical compositions containing methylphenidate in the presence of nitrite impurities is an increasing challenge for drug manufacturers. Accordingly, there is a need to develop methods to reduce nitrosamine formation in pharmaceutical compositions containing methylphenidate.

### Summary of the invention

In a first aspect, the present invention provides a pharmaceutical composition comprising methylphenidate and at least one ascorbyl fatty acid ester, wherein the molar ratio between the methylphenidate and the at least one ascorbyl fatty acid ester is from 1 : 0.1 to 1 : 5. Surprisingly, the inventors have discovered that the formation of nitrosamines in compositions with methylphenidate can be significantly reduced by using an ascorbyl fatty acid ester.

In a further aspect, the present invention relates to a method for manufacturing a pharmaceutical composition, comprising mixing of methylphenidate and at least one ascorbyl fatty acid ester, wherein the molar ratio between the methylphenidate and the at least one ascorbyl fatty acid ester is from 1 : 0.1 to 1 : 5.

In an even further aspect, the present invention relates to the pharmaceutical composition according to the first aspect for use in the treatment of Attention Deficit Disorder (ADD or ADHD).

In an even further aspect, the present invention relates to the use of an ascorbyl fatty acid ester for reducing nitrosamine formation in compositions comprising methylphenidate.

Further aspects and embodiments of the invention are disclosed in the dependent claims and can be taken from the following description, figures and examples, without being limited thereto.

### Detailed description of the present invention

### Definitions

Unless defined otherwise, technical and scientific terms used herein have the same meaning as commonly understood by one of ordinary skill in the art to which this invention belongs. Amounts within the present invention are given in wt.%, unless stated otherwise or clear from context.

Unless indicated otherwise, the term "methylphenidate" is used broadly herein to include methylphenidate and pharmaceutically acceptable salts thereof including methylphenidate hydrochloride.

As is generally known, the term "nitrosamine" refers to a class of chemical compounds characterized by the functional group R₂N-N=O, wherein R is usually an alkyl group and/or hydrogen. Nitrosamines are formed by the chemical reaction between secondary amines and nitrosating agents, such as nitrite. In the context of the present invention, the term "nitrosamine" particularly refers to N-nitrosomethylphenidate.

In the context of the present invention, a "tablet" is not particularly limited. As is generally understood, a tablet is a solid pharmaceutical dosage form that is typically prepared by compressing or molding a uniform mixture of one or more medicinally active substances and suitable excipients. A tablet is generally characterized by its solid and compact structure formed into a shape and size suitable for oral administration.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a", "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

In a first aspect, the invention relates to a pharmaceutical composition comprising methylphenidate and at least one ascorbyl fatty acid ester. The molar ratio between the methylphenidate and the at least one ascorbyl fatty acid ester is from 1 : 0.1 to 1 : 5.

In extensive studies, the inventors investigated the formation of nitrosamines in methylphenidate-containing compositions in order to find additives that could reduce or prevent this formation. Common antioxidants and stabilizers were used. Surprisingly, the inventors found that these antioxidants and stabilizers had very different levels of effectiveness in preventing nitrosamine formation. As a result, it is not possible to predict which additive will be effective in reducing the formation of nitrosamines from methylphenidate. In the studies, the inventors surprisingly found that ascorbyl fatty acid esters were the most effective in reducing nitrosamine formation. This effect is particularly pronounced at a molar ratio between the methylphenidate and the ascorbyl fatty acid ester from 1 : 0.1 to 1 : 5.

In some embodiments, the pharmaceutical composition is an oral dosage form. According to certain embodiments, the pharmaceutical composition is in solid form. According to some embodiments, the pharmaceutical composition is in tablet form. In preferred embodiments, the methylphenidate is methylphenidate hydrochloride.

According to some embodiments, at least a portion of the methylphenidate and at least a portion of the at least one ascorbyl fatty acid ester are in contact with each other. According to some embodiments, the methylphenidate and the at least one ascorbyl fatty acid ester are co-localized within the same matrix layer of the pharmaceutical composition. In certain embodiments, the pharmaceutical composition comprises or consists of a single solid phase, wherein the methylphenidate and the ascorbyl fatty acid ester are contained in said single solid phase. In this context, "single solid phase" refers to a homogenous, solid-state form wherein the methylphenidate and the ascorbyl fatty acid ester and optionally other constituents of the pharmaceutical composition such as excipients are uniformly distributed within a singular, continuous matrix. In particular, "single solid phase" does not refer to a layered structure. If the pharmaceutical composition has a layered structure, the methylphenidate and the ascorbyl fatty acid ester are in the same layer. In these embodiments, the above-mentioned effects and advantages of the present invention are particularly pronounced.

According to certain embodiments, the pharmaceutical composition further comprises at least one excipient. In some embodiments, the pharmaceutical composition is prepared by dry mixing the methylphenidate, the at least one ascorbyl fatty acid ester and optionally the at least one excipient. According to these embodiments, the above-mentioned effects and advantages of the present invention can be further improved.

In certain embodiments, the at least one ascorbyl fatty acid ester is selected from the group consisting of ascorbyl palmitate, ascorbyl stearate, ascorbyl oleate, ascorbyl linoleate, ascorbyl laurate, ascorbyl myristate, ascorbyl behenate and combinations thereof. According to preferred embodiments, the at least one ascorbyl fatty acid ester is ascorbyl palmitate. In these embodiments, the above-mentioned effects and advantages of the present invention can be further improved.

According to some embodiments, the molar ratio between the methylphenidate and the at least one ascorbyl fatty acid ester is from 1 : 0.5 to 1 : 4. In preferred embodiments, the molar ratio between the methylphenidate and the at least one ascorbyl fatty acid ester is from 1 : 1 to 1 : 3. In these embodiments, the above-mentioned effects and advantages of the present invention are particularly pronounced.

In certain embodiments, the pharmaceutical composition comprises 1.0 wt.% to 20.0 wt.%, preferably 1.5 wt.% to 15.0 wt.% and more preferably 2.0 wt.% to 13.0 wt.% methylphenidate; and 2.5 wt.% to 20.0 wt.%, preferably 5.0 wt.% to 19.5 wt.% and more preferably 10.0 wt.% to 19.0 wt.% ascorbyl fatty acid ester, based on the total weight of the pharmaceutical composition. In these embodiments, the above-mentioned effects and advantages of the present invention can be further improved.

As mentioned above, the pharmaceutical composition can further comprise at least one excipient. The excipient can be selected from the group consisting of disintegrant, binder, filler, lubricant, coloring agent and combinations thereof. In some embodiments, the pharmaceutical composition comprises a disintegrant and/or binder, a filler, a lubricant and optionally a coloring agent. According to certain embodiments, the pharmaceutical composition consists or essentially consists of methylphenidate, at least one ascorbyl fatty acid ester, a disintegrant and/or binder, a filler, a lubricant and a coloring agent. The compositions according to these embodiments can further improve the above-mentioned effects and advantages of the invention.

According to certain embodiments, the pharmaceutical composition comprises 25.0 wt.% to 35.0 wt.%, preferably 26.0 wt.% to 34.0 wt.% and more preferably 26.5 wt.% to 33.0 wt.% disintegrant and/or binder; 35.0 wt.% to 60.0 wt.%, preferably 37.0 wt.% to 57.0 wt.% and more preferably 40.0 wt.% to 55.0 wt.% filler; 0.0 wt.% to 3.0 wt.%, preferably 0.5 wt.% to 2.0 wt.% and more preferably 1.0 wt.% to 1.5 wt.% lubricant; and/or 0.0 wt.% to 2.0 wt.%, preferably 0.1 wt.% to 1.0 wt.% and more preferably 0.3 wt.% to 0.7 wt.% coloring agent, based on the total weight of the pharmaceutical composition. In some embodiments, the pharmaceutical composition consists or essentially consists of 1.0 wt.% to 20.0 wt.%, preferably 1.5 wt.% to 15.0 wt.% and more preferably 2.0 wt.% to 13.0 wt.% methylphenidate; 2.5 wt.% to 20.0 wt.%, preferably 5.0 wt.% to 19.5 wt.% and more preferably 10.0 wt.% to 19.0 wt.% ascorbyl fatty acid ester; 25.0 wt.% to 35.0 wt.%, preferably 26.0 wt.% to 34.0 wt.% and more preferably 26.5 wt.% to 33.0 wt.% disintegrant and/or binder; 35.0 wt.% to 60.0 wt.%, preferably 37.0 wt.% to 57.0 wt.% and more preferably 40.0 wt.% to 55.0 wt.% filler; 0.0 wt.% to 3.0 wt.%, preferably 0.5 wt.% to 2.0 wt.% and more preferably 1.0 wt.% to 1.5 wt.% lubricant; and 0.0 wt.% to 2.0 wt.%, preferably 0.1 wt.% to 1.0 wt.% and more preferably 0.3 wt.% to 0.7 wt.% coloring agent, based on the total weight of the pharmaceutical composition. In these embodiments, the above-mentioned effects and advantages of the present invention are particularly pronounced. If the pharmaceutical composition consists of the above-mentioned components, the percentages by weight add up to 100 wt.%, based on the total weight of the pharmaceutical composition. In this context, the phrase "essentially consists of" means that the pharmaceutical composition may contain substances in addition to the above-mentioned components, which do not materially alter the fundamental character of the pharmaceutical composition.

In some embodiments, the disintegrant and/or binder is selected from the group consisting of cellulose, maize starch and combinations thereof. Alternatively or in addition, the filler may be selected from the group consisting of calcium hydrogen phosphate, lactose and combinations thereof. Alternatively or in addition, the lubricant may be magnesium stearate. Alternatively or in addition, the coloring agent may be selected from the group of iron(III) oxide, iron(III) oxide-hydroxide and combinations thereof. In certain embodiments, the cellulose is microcrystalline cellulose. According to some embodiments, the maize starch is pregelatinised maize starch. In some embodiments, the calcium hydrogen phosphate is calcium hydrogen phosphate dehydrate. In certain embodiments, the lactose is lactose monohydrate.

According to certain embodiments, the pharmaceutical composition comprises or consists of 1.0 wt.% to 20.0 wt.%, preferably 1.5 wt.% to 15.0 wt.% and more preferably 2.0 to 13.0 wt.% methylphenidate hydrochloride; 2.5 wt.% to 20.0 wt.%, preferably 5.0 wt.% to 19.5 wt.% and more preferably 10.0 wt.% to 19.0 wt.% ascorbyl palmitate; 15.0 wt.% to 25.0 wt.%, preferably 17.0 wt.% to 24.0 wt.% and more preferably 17.5 wt.% to 23.0 wt.% cellulose; 5.0 wt.% to 15.0 wt.%, preferably 7.0 wt.% to 13.0 wt.% and more preferably 8.0 wt.% to 11.0 wt.% maize starch; 18.0 wt.% to 35.0 wt.%, preferably 20.0 wt.% to 33.0 wt.% and more preferably 23.0 wt.% to 31.0 wt.% calcium hydrogen phosphate; 13.0 wt.% to 25.0 wt.%; preferably 15.0 wt.% to 23.0 wt.% and more preferably 16.0 wt.% to 22.5 wt.% lactose; 0.0 wt.% to 3.0 wt.%, preferably 0.5 wt.% to 2.0 wt.% and more preferably 1.0 wt.% to 1.5 wt.% magnesium stearate; and 0.0 wt.% to 2.0 wt.%, preferably 0.1 wt.% to 1.0 wt.% and more preferably 0.3 wt.% to 0.7 wt.% iron(III) oxide and/or iron(III) oxide-hydroxide, based on the total weight of the pharmaceutical composition. In these embodiments, the above-mentioned effects and advantages of the present invention are further improved. If the pharmaceutical composition consists of the above-mentioned components, the percentages by weight add up to 100 wt.%, based on the total weight of the pharmaceutical composition.

In some embodiments, the pharmaceutical composition is a tablet having a methylphenidate dose of from 3 mg to 30 mg, preferably from 4 mg to 25 mg and more preferably from 5 mg to 20 mg per tablet.

In a second aspect, the invention relates to a method for manufacturing a pharmaceutical composition, wherein the method comprises mixing of methylphenidate and at least one ascorbyl fatty acid ester. The molar ratio between the methylphenidate and the at least one ascorbyl fatty acid ester is from 1 : 0.1 to 1 : 5. The pharmaceutical composition is the pharmaceutical composition according to the first aspect of the present application. In this respect, reference is made to the above statements on the first aspect of the invention, which also apply here in their entirety.

The mixing is not particularly limited. In preferred embodiments, the mixing is dry mixing. The mixing can be carried out in a free fall mixer, a tumbler mixer, a ribbon blender, a paddle mixer, a planetary mixer, a fluidized bed mixer, a cone screw mixer and/or a drum mixer. According to preferred embodiments, the mixing is carried out in a free fall mixer. In some embodiments, the mixing time is 5 minutes to 60 minutes, preferably 10 minutes to 40 minutes and more preferably 20 minutes to 30 minutes. The effect of reducing nitrosamine formation is particularly pronounced in these embodiments.

According to certain embodiments, the mixing of methylphenidate and at least one ascorbyl fatty acid ester further comprises mixing of a disintegrant and/or binder and a filler and optionally a coloring agent. In other words, the method comprises mixing methylphenidate, at least one ascorbyl fatty acid ester, a disintegrant and/or binder and a filler and optionally a coloring agent.

In some embodiments, the methylphenidate, the at least one ascorbyl fatty acid ester, the disintegrant and/or binder, the filler and optionally the coloring agent are mixed to obtain a first mixture, wherein the method further comprises mixing the first mixture and a lubricant to obtain a second mixture. The mixing of the first mixture and the lubricant is not particularly limited. In preferred embodiments, the mixing of the first mixture and the lubricant is dry mixing. The mixing of the first mixture and the lubricant can be carried out in a free fall mixer, a tumbler mixer, a ribbon blender, a paddle mixer, a planetary mixer, a fluidized bed mixer, a cone screw mixer and/or a drum mixer. According to preferred embodiments, the mixing of the first mixture and the lubricant is carried out in a free fall mixer. In some embodiments, the mixing time of mixing of the first mixture and the lubricant is 2 minutes to 40 minutes, preferably 5 minutes to 30 minutes and more preferably 10 minutes to 20 minutes.

In certain embodiments, the methylphenidate, the at least one ascorbyl fatty acid ester, the disintegrant and/or binder, the filler, the coloring agent and the lubricant are used in powder form. In preferred embodiments, the methylphenidate, the at least one ascorbyl fatty acid ester, the disintegrant and/or binder, the filler, the coloring agent and the lubricant are sieved before mixing. In particular, a sieve with a mesh size of 0.1 mm to 5.0 mm, preferably 0.3 mm to 2.0 mm and more preferably 0.4 mm to 1.2 mm can be used. In some embodiments, the calcium hydrogen phosphate and the iron oxide(s) are sieved using a sieve with a mesh size of 0.5 mm, wherein the methylphenidate, the ascorbyl fatty acid ester, the cellulose, the lactose, the maize starch and the magnesium stearate are sieved using a sieve with a mesh size of 1.0 mm. Before sieving and/or mixing, the components may be weighed according to their weight percentages (wt.%) in the pharmaceutical composition of the first aspect of the invention mentioned above. By "components" is meant methylphenidate, ascorbyl fatty acid ester and excipients.

According to some embodiments, the method further comprises compressing the second mixture to obtain tablets. The compressing is not particularly limited and can be carried out using a rotatory press, a single punch press, a hydraulic press and/or a pneumatic press. According to preferred embodiments, the compressing is carried out using a rotatory press. In certain embodiments, the compressing is performed with a compression force of 10 to 100 kN, preferably 15 to 50 kN and more preferably 20 to 30 kN.

In a third aspect, the invention relates to a pharmaceutical composition obtainable or obtained by the method according to the second aspect of the present invention. Reference is made to the above statements on the first and second aspect of the invention, which also apply here in their entirety.

In a fourth aspect, the invention relates to a pharmaceutical composition according to the first and/or third aspect for use in the treatment of Attention Deficit Disorder (ADD or ADHD). Reference is made to the above statements on the first, second and third aspect of the invention, which also apply here in their entirety.

In a fifth aspect, the invention relates to the use of an ascorbyl fatty acid ester for reducing nitrosamine formation in compositions comprising methylphenidate. In preferred embodiments, "nitrosamine" refers to N-nitrosomethylphenidate. As explained above, the inventors surprisingly found that antioxidants and stabilizers commonly used in pharmaceutical compositions had very different levels of effectiveness in preventing nitrosamine formation from methylphenidate. Therefore, it is not possible to predict which additive will be effective in reducing the formation of nitrosamines from methylphenidate. The inventors further surprisingly found that ascorbyl fatty acid esters were the most effective in reducing nitrosamine formation. In preferred embodiments, the ascorbyl fatty acid ester is ascorbyl palmitate. According to certain embodiments, the molar ratio between the methylphenidate and the at least one ascorbyl fatty acid ester is from 1 : 0.1 to 1 : 5. Reference is made to the above statements on the first, second, third and fourth aspect of the invention, which also apply here in their entirety.

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Examples

The present invention will now be described in detail with reference to several examples thereof. However, these examples are illustrative and do not limit the scope of the invention.

### Manufacturing of exemplary pharmaceutical compositions according to the present invention

An exemplary preparation of pharmaceutical compositions according to the present invention is described in the following. The amounts of the respective ingredients are shown in Table 1.

**Table 1: Exemplary manufacturing formula of pharmaceutical compositions according to the present invention**

| Entry | Ingredient | Example 1 | Example 2 | Example 3 |
|---|---|---|---|---|
| | | [kg] | | |
| 1 | Methylphenidate hydrochloride | 0.50 | 1.00 | 2.00 |
| 2 | Microcrystalline cellulose | 3.81 | 3.43 | 3.17 |
| 3 | Pregelatinised maize starch | 1.60 | 1.60 | 1.60 |
| 4 | Calcium hydrogen phosphate dihydrate | 5.22 | 4.71 | 4.23 |
| 5 | Lactose monohydrate | 3.75 | 3.37 | 3.11 |
| 6 | Magnesium stearate | 0.23 | 0.23 | 0.23 |
| 7 | Iron(III) oxide | - | 0.04 | 0.09 |
| 8 | Iron(III) oxide-hydroxide | 0.09 | 0.04 | - |
| 9 | Ascorbyl palmitate | 2.30 | 3.07 | 3.07 |

The ingredients were weighed according to the data shown in Table 1. The iron oxides and calcium hydrogen phosphate dihydrate were sieved using a sieve with a mesh size of 0.5 mm. The remaining ingredients were sieved using a sieve with a mesh size of 1.0 mm. In a first mixing step, the methylphenidate hydochloride, ascorbyl palmitate, microcristalline cellulose, lactose monohydrate, pregelatinised maize starch and iron oxides were mixed in a free fall mixer for 20 to 30 minutes. The magnesium stearate was then added to the resulting mixture and mixed in a second mixing step in the free fall mixer for 10 to 15 minutes. The resulting mixture from the second mixing step was compressed to tablets in a rotary press with a compression force of 20 to 30 kN.

The tablets of Example 1 had a methylphenidate content of 5 mg and a molar ratio of methylphenidate : ascorbyl palmitate of 1 : 3, the tablets of Example 2 had a methylphenidate content of 10 mg and a molar ratio of methylphenidate : ascorbyl palmitate of 1 : 2 and the tablets of Example 3 had a methylphenidate content of 20 mg and a molar ratio of methylphenidate : ascorbyl palmitate of 1 : 1.

### Comparison of N-nitrosomethylphenidate formation when using different oxidizing agents/stabilizers

To determine the effect of various antioxidants or stabilizers on the prevention of nitrosamine formation in compositions containing methylphenidate, different pharmaceutical compositions were prepared as described in Example 1 above, except that antioxidants/stabilizers other than ascorbyl palmitate were used. However, for better comparability, the molar ratio of methylphenidate : antioxidant/stabilizer was kept constant at 1 : 3 in all compositions (except for the composition with no antioxidant/stabilizer). Accordingly, the compositions of Example 1 and Comparative Examples 2 to 7 differ only in the type of antioxidant/stabilizer and are otherwise composed exactly as shown in Table 1 above for Example 1. The content of N-nitrosomethylphenidate in the compositions was determined by UHPLC-MS/MS. Accordingly, the term "nitrosamine" in the present context refers to "N-nitrosomethylphenidate". For sample preparation, the compositions were ground and the resulting powders were extracted with methanol. The following measurement conditions were used for the N-nitrosomethylphenidate determination:
• LC column: Waters BEH Shield RP18 (100x2.1 mm, 1.7 µm)
• Flow rate: 0.4 mL/min
• Column temperature: 50 °C
• Mobile phase:
Phase A: Water with 0.1 % by volume formic acid
Phase B: Acetonitrile

• Gradient conditions:

| Time [min] | %A | %B |
|---|---|---|
| Initial | 99.0 | 1.0 |
| 0.50 | 99.0 | 1.0 |
| 5.50 | 1.0 | 99.0 |
| 6.00 | 1.0 | 99.0 |
| 7.00 | 99.0 | 1.0 |
| 12.00 | 99.0 | 1.0 |

• Injection volume: 1 µL
• Sample temperature: Room temperature (20 °C - 22 °C)
• MS detector setting: ESI positive in MRM transition mode

The results of the determination of the N-nitrosomethylphenidate content in the compositions are shown in Table 2.

**Table 2: N-nitrosomethylphenidate formation in methylphenidate compositions with different antioxidants/stabilizers**

| Entry | Example | Antioxidant/stabilizer | Nitrosamine content [ppm] |
|---|---|---|---|
| 1 | Example 1 | Ascorbyl palmitate | 10.82 |
| 2 | Comparative Example 1 | none | 885.12 |
| 3 | Comparative Example 2 | Ascorbic acid/calcium ascorbate (buffer) | 580.48 |
| 4 | Comparative Example 3 | Butylhydroxytoluene (BHT) | 725.91 |
| 5 | Comparative Example 4 | Lysine | 77.84 |
| 6 | Comparative Example 5 | BHT/ascorbic acid buffer [1:1] | 204.59 |
| 7 | Comparative Example 6 | Lysine/ascorbic acid buffer [1:1] | 57.62 |
| 8 | Comparative Example 7 | Lysine/BHT [1:1] | 55.15 |

As can be seen from Table 2, ascorbyl palmitate is by far the most effective in reducing nitrosamine formation in compositions with methylphenidate. Surprisingly, the results for the individual antioxidants/stabilizers are very different, with common antioxidants/stabilizers such as BHT or ascorbic acid buffer only being able to prevent nitrosamine formation to a small extent. Even mixtures of several antioxidants/stabilizers, as in the Comparative Examples 5-7, are clearly not as efficient as ascorbyl palmitate in preventing the formation of N-nitrosomethylphenidate. Accordingly, ascorbyl fatty acid esters such as ascorbyl palmitate can be used effectively to reduce nitrosamine formation in compositions comprising methylphenidate.

## Claims

1. A pharmaceutical composition comprising methylphenidate and at least one ascorbyl fatty acid ester, wherein the molar ratio between the methylphenidate and the at least one ascorbyl fatty acid ester is from 1 : 0.1 to 1 : 5.

2. The pharmaceutical composition according to claim 1, wherein the at least one ascorbyl fatty acid ester is selected from the group consisting of ascorbyl palmitate, ascorbyl stearate, ascorbyl oleate, ascorbyl linoleate, ascorbyl laurate, ascorbyl myristate, ascorbyl behenate and combinations thereof, preferably wherein the at least one ascorbyl fatty acid ester is ascorbyl palmitate.

3. The pharmaceutical composition according to claim 1 or 2, wherein the molar ratio between the methylphenidate and the at least one ascorbyl fatty acid ester is from 1 : 0.5 to 1 : 4, preferably from 1 : 1 to 1 : 3.

4. The pharmaceutical composition according to any one of the preceding claims, wherein the pharmaceutical composition comprises
1.0 wt.% to 20.0 wt.% methylphenidate; and
2.5 wt.% to 20.0 wt.% ascorbyl fatty acid ester,
based on the total weight of the pharmaceutical composition.

5. The pharmaceutical composition according to any one of the preceding claims, wherein the pharmaceutical composition further comprises at least one selected from the group consisting of disintegrant, binder, filler, lubricant, coloring agent and combinations thereof, optionally wherein the pharmaceutical composition comprises a disintegrant and/or binder, a filler, a lubrincant and optionally a coloring agent.

6. The pharmaceutical composition according to any one of the preceding claims, wherein the pharmaceutical composition comprises
25.0 wt.% to 35.0 wt.% disintegrant and/or binder;
35.0 wt.% to 60.0 wt.% filler;
0.0 wt.% to 3.0 wt.% lubricant; and/or
0.0 wt.% to 2.0 wt.% coloring agent,
based on the total weight of the pharmaceutical composition.

7. The pharmaceutical composition according to any one of the preceding claims, wherein the pharmaceutical composition consists essentially of or consists of
1.0 wt.% to 20.0 wt.% methylphenidate;
2.5 wt.% to 20.0 wt.% ascorbyl fatty acid ester;
25.0 wt.% to 35.0 wt.% disintegrant and/or binder;
35.0 wt% to 60.0 wt.% filler;
0.0 wt.% to 3.0 wt.% lubricant; and
0.0 wt.% to 2.0 wt.% coloring agent,
based on the total weight of the pharmaceutical composition.

8. The pharmaceutical composition according to any one of claims 5 to 7, wherein the disintegrant and/or binder is selected from the group consisting of cellulose, maize starch and combinations thereof and/or wherein the filler is selected from the group consisting of calcium hydrogen phosphate, lactose and combinations thereof and/or wherein the lubricant is magnesium stearate and/or wherein the coloring agent is selected from the group of iron(III) oxide, iron(III) oxide-hydroxide and combinations thereof.

9. A method for manufacturing a pharmaceutical composition, comprising mixing of methylphenidate and at least one ascorbyl fatty acid ester, wherein the molar ratio between the methylphenidate and the at least one ascorbyl fatty acid ester is from 1 : 0.1 to 1 : 5.

10. The method according to claim 9, wherein the molar ratio between the methylphenidate and the at least one ascorbyl fatty acid ester is from 1 : 0.5 to 1 : 4, preferably from 1 : 1 to 1 : 3, and/or wherein the ascorbyl fatty acid ester is ascorbyl palmitate.

11. The method according to claims 9 or 10, wherein the mixing of methylphenidate and at least one ascorbyl fatty acid ester further comprises mixing of a disintegrant and/or binder and a filler and optionally a coloring agent.

12. The method according to claim 11, wherein the methylphenidate, the at least one ascorbyl fatty acid ester, the disintegrant and/or binder, the filler and optionally the coloring agent are mixed to obtain a first mixture,
wherein the method further comprises mixing the first mixture and a lubricant to obtain a second mixture,
optionally wherein the method further comprises compressing the second mixture to obtain tablets.

13. A pharmaceutical composition according to claims 1 to 8 for use in the treatment of Attention Deficit Disorder (ADD or ADHD).

14. Use of an ascorbyl fatty acid ester for reducing nitrosamine formation in compositions comprising methylphenidate.

15. The use according to claim 14, wherein the ascorbyl fatty acid ester is ascorbyl palmitate and/or wherein the molar ratio between the methylphenidate and the at least one ascorbyl fatty acid ester is from 1 : 0.1 to 1 : 5.

## Patentansprüche

1. Pharmazeutische Zusammensetzung, umfassend Methylphenidat und mindestens einen Ascorbylfettsäureester, wobei das Molverhältnis zwischen dem Methylphenidat und dem mindestens einen Ascorbylfettsäureester von 1:0,1 bis 1:5 beträgt.

2. Pharmazeutische Zusammensetzung nach Anspruch 1, wobei der mindestens eine Ascorbylfettsäureester ausgewählt ist aus der Gruppe bestehend aus Ascorbylpalmitat, Ascorbylstearat, Ascorbyloleat, Ascorbyllinoleat, Ascorbyllaurat, Ascorbylmyristat, Ascorbylbehenat und Kombinationen davon, vorzugsweise wobei der mindestens eine Ascorbylfettsäureester Ascorbylpalmitat ist.

3. Pharmazeutische Zusammensetzung nach Anspruch 1 oder 2, wobei das Molverhältnis zwischen dem Methylphenidat und dem mindestens einen Ascorbylfettsäureester von 1:0,5 bis 1:4, vorzugsweise von 1:1 bis 1:3 beträgt.

4. Pharmazeutische Zusammensetzung nach einem der vorangehenden Ansprüche, wobei die pharmazeutische Zusammensetzung umfasst:
1,0 Gew.-% bis 20,0 Gew.-% Methylphenidat; und
2,5 Gew.-% bis 20,0 Gew.-% Ascorbylfettsäureester,
bezogen auf das Gesamtgewicht der pharmazeutischen Zusammensetzung.

5. Pharmazeutische Zusammensetzung nach einem der vorangehenden Ansprüche, wobei die pharmazeutische Zusammensetzung weiterhin mindestens eines, ausgewählt aus der Gruppe bestehend aus Sprengmittel, Bindemittel, Füllstoff, Gleitmittel, Farbmittel und Kombinationen davon, umfasst, gegebenenfalls wobei die pharmazeutische Zusammensetzung ein Sprengmittel und/oder Bindemittel, einen Füllstoff, ein Gleitmittel und gegebenenfalls ein Farbmittel umfasst.

6. Pharmazeutische Zusammensetzung nach einem der vorangehenden Ansprüche, wobei die pharmazeutische Zusammensetzung umfasst:
25,0 Gew.-% bis 35,0 Gew.-% Sprengmittel und/oder Bindemittel;
35,0 Gew.-% bis 60,0 Gew.-% Füllstoff;
0,0 Gew.-% bis 3,0 Gew.-% Gleitmittel; und/oder
0,0 Gew.-% bis 2,0 Gew.-% Farbmittel,
bezogen auf das Gesamtgewicht der pharmazeutischen Zusammensetzung.

7. Pharmazeutische Zusammensetzung nach einem der vorangehenden Ansprüche, wobei die pharmazeutische Zusammensetzung im Wesentlichen besteht aus oder besteht aus:
1,0 Gew.-% bis 20,0 Gew.-% Methylphenidat;
2,5 Gew.-% bis 20,0 Gew.-% Ascorbylfettsäureester;
25,0 Gew.-% bis 35,0 Gew.-% Sprengmittel und/oder Bindemittel;
35,0 Gew.-% bis 60,0 Gew.-% Füllstoff;
0,0 Gew.-% bis 3,0 Gew.-% Gleitmittel; und
0,0 Gew.-% bis 2,0 Gew.-% Farbmittel,
bezogen auf das Gesamtgewicht der pharmazeutischen Zusammensetzung.

8. Pharmazeutische Zusammensetzung nach einem der Ansprüche 5 bis 7, wobei das Sprengmittel und/oder Bindemittel ausgewählt ist aus der Gruppe bestehend aus Cellulose, Maisstärke und Kombinationen davon und/oder wobei der Füllstoff ausgewählt ist aus der Gruppe bestehend aus Calciumhydrogenphosphat, Lactose und Kombinationen davon und/oder wobei das Gleitmittel Magnesiumstearat ist und/oder wobei das Farbmittel ausgewählt ist aus der Gruppe bestehend aus Eisen(lll)-oxid, Eisen(lll)-oxidhydroxid und Kombinationen davon.

9. Verfahren zur Herstellung einer pharmazeutischen Zusammensetzung, umfassend das Mischen von Methylphenidat und mindestens einem Ascorbylfettsäureester, wobei das Molverhältnis zwischen dem Methylphenidat und dem mindestens einen Ascorbylfettsäureester von 1:0,1 bis 1:5 beträgt.

10. Verfahren nach Anspruch 9, wobei das Molverhältnis zwischen dem Methylphenidat und dem mindestens einen Ascorbylfettsäureester von 1:0,5 bis 1:4, vorzugsweise von 1:1 bis 1:3 beträgt, und/oder wobei der Ascorbylfettsäureester Ascorbylpalmitat ist.

11. Verfahren nach den Ansprüchen 9 oder 10, wobei das Mischen von Methylphenidat und mindestens einem Ascorbylfettsäureester weiterhin das Mischen eines Sprengmittels und/oder Bindemittels und eines Füllstoffes und gegebenenfalls eines Farbmittels umfasst.

12. Verfahren nach Anspruch 11, wobei das Methylphenidat, der mindestens eine Ascorbylfettsäureester, das Sprengmittel und/oder Bindemittel, der Füllstoff und gegebenenfalls das Farbmittel gemischt werden, um ein erstes Gemisch zu erhalten,
wobei das Verfahren weiterhin das Mischen des ersten Gemischs und eines Gleitmittels, um ein zweites Gemisch zu erhalten, umfasst,
gegebenenfalls wobei das Verfahren weiterhin das Zusammenpressen des zweiten Gemischs, um Tabletten zu erhalten, umfasst.

13. Pharmazeutische Zusammensetzung nach den Ansprüchen 1 bis 8 zur Verwendung bei der Behandlung der Aufmerksamkeitsdefizitstörung (ADS oder ADHS).

14. Verwendung eines Ascorbylfettsäureesters zur Verringerung der Nitrosaminbildung in Methylphenidat umfassenden Zusammensetzungen.

15. Verwendung nach Anspruch 14, wobei der Ascorbylfettsäureester Ascorbylpalmitat ist und/oder wobei das Molverhältnis zwischen dem Methylphenidat und dem mindestens einen Ascorbylfettsäureester von 1:0,1 bis 1:5 beträgt.

## Revendications

1. Composition pharmaceutique comprenant du méthylphénidate et au moins un ester d'acide gras d'ascorbyle, dans laquelle le rapport molaire entre le méthylphénidate et l'au moins un ester d'acide gras d'ascorbyle est de 1:0,1 à 1:5.

2. Composition pharmaceutique selon la revendication 1, dans laquelle l'au moins un ester d'acide gras d'ascorbyle est choisi dans le groupe constitué par le palmitate d'ascorbyle, le stéarate d'ascorbyle, l'oléate d'ascorbyle, le linoléate d'ascorbyle, le laurate d'ascorbyle, le myristate d'ascorbyle, le béhénate d'ascorbyle et leurs combinaisons, de préférence dans laquelle l'au moins un ester d'acide gras d'ascorbyle est le palmitate d'ascorbyle.

3. Composition pharmaceutique selon la revendication 1 ou 2, dans laquelle le rapport molaire entre le méthylphénidate et l'au moins un ester d'acide gras d'ascorbyle est de 1:0,5 à 1:4, de préférence de 1:1 à 1:3.

4. Composition pharmaceutique selon l'une quelconque des revendications précédentes, dans laquelle la composition pharmaceutique comprend :
1,0 % en poids à 20,0 % en poids de méthylphénidate ; et
2,5 % en poids à 20,0 % en poids d'ester d'acide gras d'ascorbyle,
par rapport au poids total de la composition pharmaceutique.

5. Composition pharmaceutique selon l'une quelconque des revendications précédentes, dans laquelle la composition pharmaceutique comprend en outre au moins un élément choisi dans le groupe constitué par un désintégrant, un liant, une charge, un lubrifiant, un colorant et leurs combinaisons, éventuellement dans laquelle la composition pharmaceutique comprend un désintégrant et/ou un liant, une charge, un lubrifiant et éventuellement un colorant.

6. Composition pharmaceutique selon l'une quelconque des revendications précédentes, dans laquelle la composition pharmaceutique comprend :
25,0 % en poids à 35,0 % en poids de désintégrant et/ou de liant ;
35,0 % en poids à 60,0 % en poids de charge ;
0,0 % en poids à 3,0 % en poids de lubrifiant ; et/ou
0,0 % en poids à 2,0 % en poids de colorant,
par rapport au poids total de la composition pharmaceutique.

7. Composition pharmaceutique selon l'une quelconque des revendications précédentes, dans laquelle la composition pharmaceutique est essentiellement constituée ou est constituée :
de 1,0 % en poids à 20,0 % en poids de méthylphénidate ;
de 2,5 % en poids à 20,0 % en poids d'ester d'acide gras d'ascorbyle ;
de 25,0 % en poids à 35,0 % en poids de désintégrant et/ou de liant ;
de 35,0 % en poids à 60,0 % en poids de charge ;
de 0,0 % en poids à 3,0 % en poids de lubrifiant ; et
de 0,0 % en poids à 2,0 % en poids de colorant,
par rapport au poids total de la composition pharmaceutique.

8. Composition pharmaceutique selon l'une quelconque des revendications 5 à 7, dans laquelle le désintégrant et/ou le liant est choisi dans le groupe constitué par la cellulose, l'amidon de maïs et leurs combinaisons, et/ou dans laquelle la charge est choisie dans le groupe constitué par l'hydrogénophosphate de calcium, le lactose et leurs combinaisons, et/ou dans laquelle le lubrifiant est le stéarate de magnésium, et/ou dans laquelle le colorant est choisi dans le groupe constitué par l'oxyde de fer(III), l'oxyhydroxyde de fer(III) et leurs combinaisons.

9. Procédé de fabrication d'une composition pharmaceutique, comprenant le mélange de méthylphénidate et d'au moins un ester d'acide gras d'ascorbyle, dans lequel le rapport molaire entre le méthylphénidate et l'au moins un ester d'acide gras d'ascorbyle est de 1:0,1 à 1:5.

10. Procédé selon la revendication 9, dans lequel le rapport molaire entre le méthylphénidate et l'au moins un ester d'acide gras d'ascorbyle est de 1:0,5 à 1:4, de préférence de 1:1 à 1:3, et/ou dans lequel l'ester d'acide gras d'ascorbyle est le palmitate d'ascorbyle.

11. Procédé selon la revendication 9 ou 10, dans lequel le mélange de méthylphénidate et d'au moins un ester d'acide gras d'ascorbyle comprend en outre le mélange d'un désintégrant et/ou d'un liant et d'une charge, et éventuellement d'un colorant.

12. Procédé selon la revendication 11, dans lequel le méthylphénidate, l'au moins un ester d'acide gras d'ascorbyle, le désintégrant et/ou le liant, la charge et éventuellement le colorant sont mélangés afin d'obtenir un premier mélange,
dans lequel le procédé comprend en outre le mélange du premier mélange et d'un lubrifiant afin d'obtenir un deuxième mélange,
éventuellement dans lequel le procédé comprend en outre la compression du deuxième mélange afin d'obtenir des comprimés.

13. Composition pharmaceutique selon les revendications 1 à 8 destinée à être utilisée dans le traitement du trouble déficitaire de l'attention (TDA ou TDAH).

14. Utilisation d'un ester d'acide gras d'ascorbyle pour réduire la formation de nitrosamines dans des compositions comprenant du méthylphénidate.

15. Utilisation selon la revendication 14, dans laquelle l'ester d'acide gras d'ascorbyle est le palmitate d'ascorbyle et/ou dans laquelle le rapport molaire entre le méthylphénidate et l'au moins un ester d'acide gras d'ascorbyle est de 1:0,1 à 1:5.
